# EUROPEAN PATENT APPLICATION

(11) **EP 1 530 968 A1**
(43) Date of publication of application: **18.05.2005**
(21) Application number: 03078578.6
(22) Date of filing: 14.11.2003
(51) Int. Cl.: A61K 31/352, A61P 19/10, A61P 19/00

(54) **Galangin components for the maintenance of bone health and/or treatment, alleviaton and/or prevention of bone disorder**

(71) Applicant: N.V. Nutricia, 2712 HM Zoetermeer (NL)
(72) Inventor: Schoenmakers, Ines, 3514 XJ Utecht (NL); Gros-Van Hest, Marjan, 2300 RC Leiden (NL); Löwik, Clemens Waltherus Gerardus Maria, 2343 DC Oegstgeest (NL)
(74) Representative: van Westenbrugge, Andries

(57) **Abstract**

The present invention relates to a method for the maintenance of bone health and/or the treatment, alleviation and/or prevention of bone disorder, said method comprising the administration to a mammal of an effective amount of galangin, a pharmaceutically acceptable salt of galangin or a precursor of galangin in an amount equivalent to between 0.5 and 100 mg galangin per kg body weight per day.

## Description

### FIELD OF THE INVENTION

The present invention relates to a method for the maintenance of bone health or the treatment, alleviation and/or prevention of bone disorder, said method comprising the enteral or parenteral administration of flavonoids in an effective amount.

### BACKGROUND OF THE INVENTION

Bone loss occurs in many diseases such as osteoporosis, Paget's disease, osteolytic metastasis in cancer patients and osteodystrophy in liver disease.

The most common metabolic bone disorder is osteoporosis. Osteoporosis is a crippling disease that emerges as an important public health problem in developed as well as developing countries. The clinical condition of osteoporosis is characterized mainly by loss of bone mineral mass, rendering bones more fragile and susceptible to fracture. In humans, osteoporosis is a common feature of aging.

Hormone replacement therapy (HRT) is an effective method to prevent and treat estrogen deficiency induced bone loss. HRT is not an optimal therapy since only post-menopausal women can be treated with HRT, and HRT may increase the risk on breast cancer.

Phyto-oestrogenic plant components and several flavonoids (e.g. kaempherol), have also been suggested for use in a method for the treatment or prevention of bone loss (JP2003026572, US5478579). However these components have strong estrogenic action resulting in undesired side effects. It is believed that components with estrogenic action may increase the risk on breast cancer. Hence, these components are not optimal for the treatment or prevention of bone loss.

### SUMMARY OF THE INVENTION

The present inventors have surprisingly found that galangin can be advantageously used in a method for the maintenance of bone health or the treatment, alleviation and/or prevention of bone disorder, preferably in a method for the treatment or prevention of a bone loss inducing condition and/or stimulating bone formation in mammals.

It was found that galangin simulates calcium deposition and stimulates alkaline phosphatase, indicating that galangin is capable of stimulating extracellular matrix formation and calcification (e.g. stimulating bone formation) and is capable of increasing osteoblast activity. It was also found that galangin is capable of inhibiting bone loss in mammals. Hence, galangin can be advantageously used in a method for maintenance of bone health or the treatment, alleviation and/or prevention of bone disorder.
As an improvement over the use of other bioflavonoids, it was also found that galangin, or galangin component (see below), in contrast to many other flavonoids, has only very mild estrogenic action, i.e. stimulates the estrogen receptor (ER receptor) only very weakly while it is a strong activator of the bone morphogenetic protein receptor (BMP-receptor). Bone morphogenetic proteins (BMP's) regulate and stimulate osteoblast differentiation and activity. Hence, galangin has reduced (estrogenic) side effect, which makes the present galangin component particularly suitable for the treatment and/or prevention of bone disorder and/or for the maintenance of bone health.

As a further advantage, the galangin is only a weak pro-oxidant as compared to other flavonoids suggested for the treatment or prevention of bone loss inducing conditions. The galangin component is more stable in a nutritional composition and causes less oxidative damage in the body as compared to many other flavonoids. The galangin component can be advantageously added to a nutritional composition comprising carbohydrate, protein and fat. Such nutritional composition is particularly suitable for elderly humans, as this patient population is in need of increased bone strength and reduced bone loss.

### DETAILED DESCRIPTION OF THE PREFERRED EMBODIMENTS

The present invention provides a method for the treatment or prevention of a bone loss inducing condition and/or stimulating bone formation in mammals, said method comprising administering to the mammal a galangin component selected from the group consisting of galangin, a pharmaceutically acceptable salt of galangin and a precursor of galangin in an amount equivalent to between 0.05 and 100 mg galangin per kg body weight per day.

In a further aspect the present invention provides a unit dosage comprising
a. a galangin component selected from the group consisting of galangin, a pharmaceutically acceptable salt of galangin and a precursor of galangin in an amount equivalent to between 2 mg and 5 g galangin;
b. between 25 mg and 5 g calcium; and
c. between 1 µg and 1000 µg Vitamin D

Still a further aspect of the present invention relates to an enteral preparation comprising between 5 and 50 en% lipid, between 10-60 en% protein, between 15-90 en% carbohydrate and a galangin component selected from the group consisting of galangin, a pharmaceutically acceptable salt of galangin and a precursor of galangin in an amount equivalent to between 2 mg and 5 g galangin per serving, or alternatively in an amount equivalent to between 10 mg and 5 g galangin per liter (en% is short for energy percentage and represents the relative amount each constituent contributes to the total caloric value of the preparation).

### Chemical structure

The component of the present invention is a galangin component selected from the group consisting of galangin, a pharmaceutically acceptable salt of galangin and a precursor of galangin. The structure of galangin (3,5,7-trihydroxyflavone) is given in formula I.

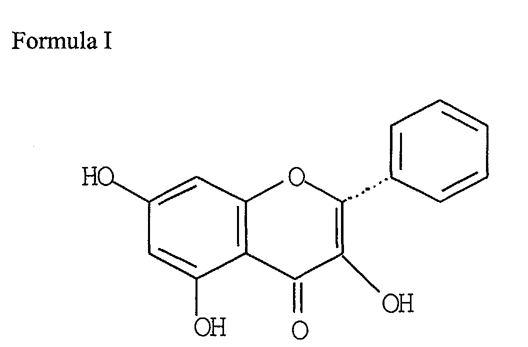

The group consisting of galangin, a pharmaceutically acceptable salt of galangin and a precursor of galangin is hereinafter referred to as " galangin component". Preferably, galangin or a pharmaceutically acceptable salt of galangin is used. Most preferably galangin is used.

Precursors of galangin are compounds that can be converted into galangin by gastrointestinal hydrolytic cleavage, such as for example carbohydrate derivatives, carbonate derivatives, ester derivatives and ether derivatives. In an embodiment, the precursors of the galangin component are selected from the group consisting of glycosides, rutinosides, glucuronoside, gentobioside and methyl ethers, in particular glycosides of galangin and 3-methyl galangin.

### Unit Dosages

The present method preferably comprises the administration of galangin component in a daily dosage containing the equivalence of between 0.05 and 100 mg, preferably between 0.25 and 25 mg, even more preferably between 1 and 10 mg galangin per kg body weight.

Unit dosages, which can be suitably used in the present method, preferably comprise the equivalence of between 1 mg and 2.5 g, preferably between 2.5 mg and 1 g, even more preferably between 10 mg and 500 mg galangin, e.g. between 1 mg and 2.5 g, preferably between 2.5 mg and 1 g, even more preferably between 10 mg and 500 mg galangin component. These unit dosages are particularly suitable for humans.

Advantageously, the present method comprises the administration of a dosage containing at least 1 wt.%, more preferably at least 5 wt.%, even more preferably at least 10 wt.%, most preferably at least 25 wt.% galangin component based on the total dry weight of the dosage.

To reduce dosage size, the galangin component is preferably provided by a galangin component containing raw material (e.g. a plant extract) containing at least 10 wt.%, preferably 25 wt.%, more preferably at least 50 wt.%, even more preferably at least 90 wt.% galangin component based the dry weight of the galangin component containing raw material.

### Sources and purity of component

In a particularly preferred embodiment, the galangin component, preferably galangin, is provided by a galangin component containing plant material, preferably in the form of a plant isolate thereof. The term "plant isolate" as referred to in here, encompasses any fraction obtained from a plant material by means of isolation techniques known in the art, e.g. extraction, distillation, squeezing etc. and that has an increased flavonoid content compared to the dry plant raw material. Preferably the plant material or plant isolate contains at least 1 wt.%, more preferably at least 5 wt.%, even more preferably at least 10 wt.%, most preferably at least 50 wt.% galangin component based on the total dry weight of the plant material/isolate. Usually, the concentration of the galangin component of the plant material or isolate does not need to exceed 99.5 wt.% based on the total dry weight of the plant material or isolate.

Preferably the galangin component is derived from plant material which, by its nature, has a relatively high content of galangin component. Suitable sources of galangin component include Alpina species (e.g *Alpinia galanga* or *Alpinia officinarum* Hance), Glycyrrhiza and Populus. Propolis may also be used a source of galangin component, however less suitable for administration as its use may result in severe allergy reactions or even anaphylactic shock.

### Treatment

The present invention relates to a method for maintenance of bone health and/or the treatment, alleviation and/or prevention of bone disorder, preferably to a method for the treatment or prevention of bone loss inducing condition and/or stimulating bone formation in mammals, particularly to a method for the treatment or prevention of bone loss inducing condition. It is believed that the galangin component has an optimal effect in sub-acute or chronic bone loss inducing conditions, as the effects of the galangin component are optimal if it is administered over a longer period of time, e.g. for at least 10 consecutive days, more preferably for at least 25 consecutive days.
Conditions which can be suitably treated with the galangin component are selected from the group consisting of osteoporosis, Paget's disease, osteolytic metastasis in cancer patients, osteodystrophy in liver disease, altered bone metabolism caused by renal failure or haemadialysis, bone surgery, pregnancy, lactation, anorexia nervosa, immobility, malnourishment and weight reduction. The galangin component is particularly suitable for the prophylactic and/or curative treatment of osteoporosis.

The galangin component may also be suitably employed in packaged nutritional supplements, which have been provided with labels that explicitly or implicitly direct the consumer towards the use of said supplement or product in accordance with one or more of the above or below described purposes. Such labels may for example make reference to bone loss related conditions by incorporation of terminology like "enhanced bone strength", "bone health", "OsteoOptimal", "Bone Formula", "OsteoProCare™" and the like. The bone condition improving properties of the product may be indicated via indicia such as pictures, drawings and other indicia from which a consumer can conclude that the product aims to treat or prevent bone loss inducing conditions.

### Administration as a unit dosage

According to a preferred embodiment the present method comprises the oral administration of the galangin component to a mammal. Preferably, the galangin component is administered as a unit dosage. The unit dosage used in the present method can be applied in any suitable form, such as bars, pills, capsules, gels, liquid etc, however is preferably provided in the form of a pill, tablet, caplet or capsule. Preferably a unit dosage does not consist of more than 3 tablets, caplets, capsules or pills, even more preferably consists of a single pill, capsule, caplet or tablet. Advantageously, at least two unit dosages, more preferably at least three unit dosages, are administered in one day. In the present method a daily dosage of the preparation as used in the present invention can include one or more pills, tablets or capsules. Preferably a daily dosage consists of 1 to 6 pills, tablets, caplets or capsules.
The present unit dosage is preferably in a solid or semisolid form, more preferably in a form selected from the group consisting of pills, capsules, tablets, caplet, microparticles and microspheres. The solid or semisolid dosage form preferably has a weight between 0.1 and 30 g, more preferably between 0.2 and 10 g.
The unit dosage preferably has a weight between 0.2 and 4 g, even more preferably between 0.5 and 3 g.

### Administration in a nutritional composition

The present invention also relates to a nutritional composition comprising between 5 and 50 en% lipid, between 10 and 60 en% protein, between 15 and 90 en% carbohydrate and a galangin component selected from the group consisting of galangin, a pharmaceutically acceptable salt of galangin and a precursor of galangin in an amount equivalent to between 2 mg and 5 g galangin per serving. The present nutritional composition preferably contains between 10 and 30 en% lipid, between 15 and 40 en% protein and between 25 and 75 en% carbohydrate.
Preferably vegetable lipids are used. The vegetable lipid is preferably at least one selected from the group consisting of soy oil, palm oil, coconut oil, safflower oil, sunflower oil, corn oil, canola oil and lecithins. Animal fats such as milk fats may also be added if desired.
The proteins used in the nutritional preparation are preferably animal proteins (such as milk proteins, meat proteins and egg proteins); vegetable proteins (such as soy protein, wheat protein, rice protein, and pea protein); mixtures of free amino acids or combinations thereof. Milk proteins such as casein, whey proteins and soy proteins are particularly preferred.
A source of carbohydrate may be added to the nutritional formula. It preferably provides about 40% to about 80% of the energy of the nutritional composition. Any suitable carbohydrates may be used, for example sucrose, lactose, glucose, fructose, corn syrup solids, and maltodextrins, and mixtures thereof. Dietary fiber may also be added if desired.

A serving of the present nutritional composition is mostly between 50 and 1500 ml, preferably between 100 and 500 ml. The nutritional composition preferably contains an amount equivalent to between 10 mg and 1 g, even more preferably between 25 mg and 500 mg galangin, most preferably between 50 and 250 mg galangin per serving. The nutritional composition suitably contains between 10 mg and 5 g galangin component per liter, preferably between 25 mg and 1 g galangin component per liter. In an embodiment the nutritional composition comprises further between 100 mg and 5 g calcium; and between 0.1 µg and 100 µg Vitamin D per liter.
The galangin component containing nutritional composition can be suitably used in a method for the treatment or prevention of loss inducing condition and/or stimulating bone formation in mammals, said method comprising the enteral administration to the mammal of said nutritional composition.
For optimal treatment of the elderly, the present nutritional composition preferably has a caloric density of between 0.5 and 2.5 kcal per ml.

### Combinations

The present invention also provides compositions that are suitable for maintaining bone health and/or for use in the treatment, alleviation and/or prevention of bone disorder, e.g. for use in the treatment or prevention of a bone loss inducing condition and/or stimulating bone formation, particularly osteoporosis, said composition comprising galangin component and an effective amount of calcium. It was found that galangin component stimulates and accelerates bone formation. To prevent shortages in calcium availability, calcium is preferably coadministered with the galangin component and the combination is deemed to synergistically treat and/or prevent the bone loss inducing disorders, e.g. osteoporosis.

Preferably the present unit dosage comprises between 100 mg and 5 g, even more preferably between 250 mg and 2.5 g calcium. The present nutritional composition preferably contains between 50 mg and 2 g calcium per liter, more preferably between 100 mg and 1 g calcium per liter.
A method for the treatment or prevention of one or more of the above-decribed disorders preferably comprises the daily administration of between 10 mg and 10 g, preferably between 60 mg and 1 g, even more preferably between 80 mg and 800 g calcium.

Zinc can be suitably coadministered in an amount up to 40 mg per day, preferably not exceeding 30 mg per day. The zinc content is preferably kept below 15 mg per serving to avoid adverse taste effects.

In a further embodiment the invention provides compositions that are suitable for use in the treatment or prevention of a bone loss inducing condition and/or stimulating bone formation, particularly osteoporosis, said composition comprising galangin component and an effective amount of vitamin D. Even more preferably, such composition also comprises calcium in the above give amounts. Such compositions are advantageously used in the present method. It is the inventors believe that galangin component, vitamin D and calcium synergistically improve bone formation.

Preferably the present unit dosage comprises between 0.01 µg and 1000 µg vitamin D, even more preferably between 0.1 µg and 100 µg vitamin D. The present nutritional composition preferably comprises between 0.1 and 100 µg vitamin D per liter, even more preferably between 1 and 50 µg vitamin D per liter. The present method preferably comprises the daily administration of between 0.1 and 100 µg, more preferably between 0.5 and 50 µg vitamin D per day.

In a further embodiment, the galangin component is preferably coadministered with a flavonoid other than galangin and/or isoflavone. Preferably the flavonoid other than galangin is selected from the group consisting of luteolin, morin, apigenin, kaemferol and quercitin. The isoflavone is preferably selected from the group consisting of genistein, genestin, daizin and daidzein. As different receptors are involved in the simulation of bone strength and/or prevention of bone loss, the coadministration of galangin with a flavonoid other than galangin and/or galangin with an isoflavone, or both, is deemed to provide a synergistic effect and/or to reduce the oestrogenic effects which would be experienced when the flavonoid other than galangin and/or isoflavone were administered without the galangin component.

The flavonoids other than galangin are preferably administered in an amount of between 0.1 mg and 1 g, preferably between 5 mg and 700 mg, even more preferably between 25 and 500 mg per day.
The isoflavone or mixture of isoflavones is preferably administered in an amount of between 0.02 and 10 µmol, preferably between 0.1 and 5 µmol per kg body weight of the subject per day.

### EXAMPLES

### Example 1: Alkaline phosphatase and calcium deposition by galangin

KS483 (mouse pre-osteoblastic cell line, Leids Universitair Medisch Centrum, Leiden, The Netherlands) was maintained in phenol red-free alpha-minimum essential medium (α-MEM) containing 10% non-charcoal-treated fetal bovine serum (FBS) and penicillin/streptomycin. Cells were seeded in 12-well plates at a density of 4.5x10⁴ cells/well, and cultured in phenol red-free α-MEM supplemented with penicillin/streptomycin and 10% charcoal-treated FBS. Cells were exposed to vehicle (0.01 wt.% DMSO) , β-estradiol as a positive control (10⁻⁸ M and 10⁻⁹ M in 0.01 wt.% DMSO) or galangin (10⁻⁵ M, 10⁻⁶ M and 10⁻⁷ M in 0.01 wt.% DMSO) for 18 days. From day 4 ascorbic acid (50µg/ml), and from day 11 β-glycerolphosphate (5mM) were added to the culture medium. Every 3-4 days the medium was changed.

At day 18, cells were washed twice with ice-cold phosphate buffered saline (PBS), frozen immediately, and stored at -20°C for further use. The cells were lysed in 400µl SSC (15 mM sodium citrate and 150 mM sodium chloride) with 0.01% sodium dodecyl sulfate (SDS). Alkaline phosphatase (ALP) activity was determined kinetically in 25µl aliquots by colorimetric determination of the produced p-nitrophenol at 405 nm, using 6 mmol/L p-nitrophenylphosphate as a substrate at a 37°C and pH 10.5. To correct the ALP activity determination for cell quantity, the DNA content was measured in 100µl aliquots, using Hoechst 33258 and calibrated against a DNA standard (0.5-10µg/mL salmon sperm DNA). Fluorescence was measured at a wavelength of 355-460 nm. The ALP activity (corrected for DNA) is given in Table 1.
Calcium deposition (see Table 2) was determined using the cresolphthalein complexon method according to Henri et al, 1974 (Clinical Biochemistry. Principles and Techniques. Harper and Row, New York, USA. All chemicals were obtained from Sigma-Aldrich (Zwijndrecht, The Netherlands).

**TABLE 1**

| ALP activity (corrected for DNA) expressed as fold induction compared to control levels. | |
|---|---|
| Component | ALP activity (mean ± SEM) |
| control | 1.0 ± 0.026 |
| β-E 10⁻⁹M | 2.5 ± 0.184^{a} |
| β-E 10⁻⁸M | 2.6 ± 0.129^{a} |
| Gal 10⁻⁷M | 1.1 ± 0.071 |
| Gal 10⁻⁶M | 1.4 ± 0.055^{a} |
| Gal 10⁻⁵M | 3.7 ± 0.152^{a} |

| | |
|---|---|
| ^{a} p<0.001 | |

**TABLE 2**

| Calcium deposition expressed as fold induction compared to control levels. | |
|---|---|
| Component | Fold increase of calcium (mean ± SEM) |
| control | 1.0 ± 0.093 |
| β-E 10⁻⁹M | 1.3 ± 0.064 |
| β-E 10⁻⁸M | 1.9 ± 0.120^{a} |
| Gal 10⁻⁷M | 1.3 ± 0.038 |
| Gal 10⁻⁶M | 1.2 ± 0.132 |
| Gal 10⁻⁵M | 2.3 ± 0.160^{a} |

| | |
|---|---|
| ^{a} p<0.001 | |

Galangin significantly increased ALP activity in KS483 cells, which in indicative for the suitability of the galangin component for use in the present method. Calcium deposition was also significantly increased after treatment with galangin, which is a further indication for the suitability of the galangin component for use in the present method.

### Example 2: ERE/BRE ratio of galangin and other flavonoids

Mouse pre-osteoblastic cell line KS483 was maintained in phenol red-free α-MEM containing 10% non-charcoal-treated FBS and penicillin/streptomycin. Cells were seeded in 24-well plates at a density of 1.6x10⁴ cells/well, and cultured in phenol red-free α-MEM supplemented with penicillin/streptomycin and 10% charcoal-treated FBS. After 24 hours, cells were transiently transfected with 0.25 µg luciferase reporter (see below) using a lipid-based FuGENE 6 transfection reagent according to the manufacturer's recommendations (Roche, Almere, Netherlands). Cells were transfected with luciferase reporter, i.e. either estrogen-receptor-responsive element (ERE-Luciferase) or BMP-receptor-responsive element (BRE-Luciferas), hereinafter jointly referred to as luciferase reporter. Transfection efficiency was measured by co-transfection of 25 ng of SV40-Renilla control vector.. The transfection medium was changed after 18 hours into new α-MEM, containing penicillin/streptomycin and 10% charcoal-treated FBS. Thereafter, cells were exposed to vehicle (0.01 wt.% DMSO in water) or different concentrations of β-estradiol ( E), galangin (Gal), quercetin (Q), kaemferol (K), Bone Morphogenetic Protein (BMP) in 0.01 wt.% DMSO in water. After 48h, cells were washed twice with PBS and frozen at -20°C. Luciferase activity was determined by measuring bioluminescence. Luciferase activity was expressed as fold induction ± SEM, which was corrected for transfection efficiency using sv40-renilla luciferase activity. The measured luciferase activity is indicative for the extent of stimulation of the estrogen receptor or BMP receptor by the test component. Subsequently the ratio ERE stimulation/BRE stimulation (ERE/BRE ratio) was determined, which is indicative for the estrogenic effects of the test component (Table 3).

**TABLE 3**

| Stimulation of estrogen receptor (ERE) and BMP receptor (BRE) by test components and ratio ERE stimulation/BRE stimulation of test components. | | | |
|---|---|---|---|
| | ERE | BRE | ERE/BRE |
| control | 1.0 | 1.0 | 1.0 |
| BMP25 | 1.8 | 6.9 | 0.3 |
| BMP50 | 2.8 | 8.8 | 0.3 |
| Gal10-7M | 0.9 | 0.9 | 1.1 |
| Gal10-6M | 1.0 | 0.9 | 1.2 |
| Gal10-5M | 3.8 | 2.0 | 2.0 |
| K10-7M | 1.2 | 1.1 | 1.1 |
| K10-6M | 5.9 | 1.4 | 4.1 |
| K10-5M | 13.7 | 2.4 | 5.7 |
| Q10-7M | 1.4 | 0.9 | 1.6 |
| Q10-6M | 1.5 | 1.0 | 1.6 |
| Q10-5M | 7.9 | 1.6 | 5.0 |
| βE 10-10M | 20.2 | 1.0 | 19.4 |
| βE 10-9M | 15.8 | 1.6 | 10.2 |
| βE 10-8M | 9.0 | 1.0 | 8.6 |

Obviously, -estradiol has a high ERE/BRE ratio, as it is known to have strong estrogenic effects. BMP has a very low ERE/BRE ratio, as BMP is known to selectively stimulate the BMP receptor.
It was surprisingly found that galangin has a low ERE/BRE ratio as compared to kaempherol and quercetin. The ratio ERE/BRE of galangin was found to less than about 50% of the kaempherol and quercitin.

The low ERE/BRE ratio of galangin is indicative for the weak estrogenic effects of galangin component, and its suitability for use in a method for the treatment of chronic bone loss.

### Example 3: Bone density increase in mice.

Senescence accelerated mice prone for osteoporosis (SAMP6) and senescence accelerated resistant mice (SAMR) were obtained from Kyoto University, Kyoto, Japan. Mice were bred and kept under specific pathogen free conditions at the Center for small experimental animals in Wageningen. Procedures were approved by the external independent ethical committee for animal care and use (Zeist, The Netherlands). Female SAMP6 mice were randomized on the basis of body weight at the age 20 weeks and divided in 5 groups. 16 mice were designated as controls (SAMP6-C) and 4 groups of 8 mice each were treated with respectively, subcutaneous 17 β-Estradiol implants as a positive control (SAMP6- E), and diets enriched with three different concentrations of galangin (0.4, 2 and 20 mg galangin per 100 g feed; SAMP6-Ga10.4, SAMP6-Gal2 and SAMP6-Ga120 respectively). The galangin was obtained from ICC, Sommerville, NJ, USA. The mice were treated from the age of 22 weeks to the age of 32 weeks. Animals were housed in pairs at 21°C with a 12-hour light/dark cycle in a room without male mice.
Until the age of 22 weeks, animals were fed a complete rodent diet, free of flavonoids and isoflavones (RMH-B, Hope Farms, Woerden, The Netherlands). From 22 to 32 weeks of age, animals were fed diets composed according to the RDA of mice (Nutrient requirements of mice, National Academic press, Wahington, USA,1995) and demineralized water. Composition of all diets was identical, except for their galangin contents. All diets and water were supplied ad libitum. Food and water intake and body weight (BW) was registered weekly. Animals were checked daily for abnormal behavior and appearance.
The sub-cutenous β-Estradiol implants were constructed of silicon tubes filled with 2 mg of a 1:4 mixture of 17 β-estradiol and cholesterol (Sigma-Aldrich, Zwijndrecht, The Netherlands). Implants were replaced after 6 weeks.

At the age of 22, 25, 28 and 32 weeks, bone mineral density (BMD) and body composition was determined with the aid of Dual energy X-ray absorptiometry (DXA) (Piximus, Lunar Corp, Madison, WI, USA) using the total body mode. Mice were anaesthetized with halothane and front and hind legs were extended. Bone mineral density of the lumbar spine (BMDLS) and the femur (BMDF) were determined within pre-defined regions of interest. Total body fat percentage was determined using the total body mode.

### Results

No abnormal behavior or appearance of the animals was observed during the entire research period, except for one animal in the control group. This animal was excluded from the experiment due to illness unrelated to the treatment.
No statistical differences were present in body weight, fat percentage, food and water intake between control and galangin-treated mice throughout the study. For the animals treated with E, body weight, fat percentage, food and water intake was significantly lower than in the control group from 25 weeks of age onwards.
Uterus weight at 32 weeks of age was comparable among control and galangin treated groups, but significantly higher in the E treated group.
BMDLS and BMDF were comparable between all groups before the start of the intervention (Table 4, week 22). At 32 weeks, the group receiving 0.4 mg galangin/100 g feed had a significantly higher BMD-LS than BMDC . The loss of BMD-LS during the 10-week intervention period was significantly lower in the mice receiving 0.04 mg galangin/100 g food than in controls.

The dosage of 17 β-Estradiol applied, appeared to be supra-physiological when used in intact SAMP6-mice. This led to a significantly higher BMD-LS and BMD-F from 25 weeks of age onwards.

**TABLE 4**

| Bone mineral density (g/cm²) of the lumbar spine (BMD-LS) and BMD-LS change ( BMDLS) after 10 weeks of treatment (mean ± SEM) | | | | | | |
|---|---|---|---|---|---|---|
| | Age (weeks) | | | | | |
| | 22 | 25 | 28 | 32 | BMDLS | % inhibition compared to SAMPC |
| SAMR | 0.0829 | 0.0738 | 0.0701 | 0.0751 ^{a} | -12.4 ^{a} | |
| SAMP6-C | 0.0710 | 0.0610 | 0.0588 | 0.0545 | -23.1 ^{a} | 100% |
| SAMP6- E | 0.0700 | 0.0774 | 0.08315 | 0.0868 ^{a} | +23.4 ^{a} | |
| SAMP6-Ga10.4 | 0.0691 | 0.0658 | 0.0621 | 0.0601 ^{a} | -14.1 ^{a} | 40% |

| | | | | | | |
|---|---|---|---|---|---|---|
| ^{a}: significantly different (P < 0.05) when compared to SAMPC | | | | | | |

The bone mass density stimulatory effects of galangin are indicative for the advantageous use of galangin in the present method and composition.

### Example 4: Unit dosage

Packaged unit dosages, said package containing a label which indicates that the unit dosages can be orally ingested by elderly humans who are in need or desire to stimulate bone strength or reduce bone loss, said unit dosages comprising 50 mg galangin, 200 mg calcium, 200 IU vitamin D and 300 mg pharmaceutically acceptable excipient.

### Example 5: Nutritional composition for elderly humans

The enteral nutritional composition of the present invention preferably has the following nutrient composition (per 1200 ml of a composition comprising 1 kcal/ml):

| Nutrient Composition | Amount |
|---|---|
| Protein | 54 g |
| Carbohydrate | 156 g |
| Fat | 40.6 g |
| Dietary Fiber | 10 g |
| Water | 742 ml |
| Vitamin A | 4000 IU |
| Beta-Carotene | 6 mg |
| Vitamin D | 600 IU |
| Vitamin E | 100 IU |
| Vitamin K | 80 mcg |
| Vitamin C | 240 mg |
| Thiamine (B₁) | 2.25 mg |
| Riboflavin (B₂) | 2.55 mg |
| Niacin | 40 mg |
| Vitamin B₆ | 4 mg |
| Folic Acid | 1200 mcg |
| Pantoth Acid | 15 mg |
| Vitamin B₁₂ | 12 mcg |
| Biotin | 400 mcg |
| Choline | 452 mg |
| Taurine | 100 mg |
| Camitine | 100 mg |
| Calcium | 1250 mg |
| Phosphorus | 1000 mg |
| Magnesium | 400 mg |
| Zinc | 24 mg |
| Iron | 18 mg |
| Copper | 2 mg |
| Magnesium | 4 mg |
| Iodine | 150 mcg |
| Sodium | 763 mg |
| Potassium | 1560 mg |
| Chloride | 1296 mg |
| Chromium | 100 mcg |
| Molybdemum | 150 mcg |
| Selenium | 80 mcg |
| Galangin | 35 mg |

## Claims

1. Use of galangin component in the manufacture of a composition for use in a method for the maintenance of bone health and/or the treatment, alleviation and/or prevention of bone disorder in mammals, said method comprising administering to the mammal a galangin component selected from the group consisting of galangin, a pharmaceutically acceptable salt of galangin and a precursor of galangin in an amount equivalent to between 0.05 and 100 mg galangin per kg body weight per day.

2. Use according to claim 1, wherein said method comprises the treatment or prevention a sub-acute or chronic bone loss inducing condition and/or stimulating bone formation in mammals.

3. Use according to claim 2, wherein the condition is selected from the of selected from the group consisting of osteoporosis, Paget's disease, osteolytic metastasis in cancer patients, osteodystrophy in liver disease osteoporosis, altered bone metabolism caused by renal failure or haemadialysis, bone surgery, pregnancy, lactation, anorexia nervosa, immobility, malnourishment and weight reduction.

4. Use according to claim 1, for the treatment or prevention of osteoporosis.

5. Use according to any one of the preceding claims, wherein the method comprises the oral administration a unit dosage comprising at least 1 wt.% of said galangin component based on the total dry weight of the unit dosage.

6. Use according to any one of the preceding claims, wherein the method further comprises the coadministration of between 10 mg and 10 g calcium per day.

7. Use according to any one of the preceding claims, wherein the method further comprises the coadministration of between 0.1µg and 100 µg vitamin D per day.

8. Use according to any one of the preceding claims, wherein the mammal is a human.

9. Use according to any one of the preceding claimss, wherein the mammal is an elderly human.

10. A unit dosage comprising
a. a galangin component selected from the group consisting of galangin, a pharmaceutically acceptable salt of galangin and a precursor of galangin in an amount equivalent to between 2 mg and 5 g galangin;
b. between 25 mg and 5 g calcium; and
c. between 0.01 µg and 1000 µg Vitamin D

11. An enteral preparation comprising between 5 and 50 en% lipid, between 10 and 60 en% protein, between 15 and 90 en% carbohydrate and a galangin component in an amount equivalent to between 2 mg and 5 g galangin per serving, wherein said galangin component is selected from the group consisting of galangin, a pharmaceutically acceptable salt of galangin and a precursor of galangin.

12. An enteral preparation according to claim 11, further comprising between 100 mg and 5 g calcium; and between 0.1 µg and 100 µg Vitamin D per liter.

13. Use of an the enteral composition according to any one of claims 10-12 in the manufacture of a composition for use in a method for the maintenance of bone health and/or the treatment, alleviation and/or prevention of bone disorder in mammals, said method comprising administering the enteral administration to the mammal the composition of claim 10-12.
